# EUROPEAN PATENT APPLICATION

(11) **EP 1 101 495 A1**
(43) Date of publication of application: **23.05.2001**
(21) Application number: 00929921.5
(22) Date of filing: 30.05.2000
(51) Int. Cl.: A61K 45/00, A61K 39/395, A61P 7/04

(54) **PREVENTIVES FOR IDIOPATHIC THROMBOCYTOPENIC PURPURA**

(30) Priority: 01.06.1999 JP 15338399
(71) Applicant: Eisai Co., Ltd., Tokyo 112-8088 (JP)
(72) Inventor: YAMAUCHI, Toshihiko, Tsukuba-shi, Ibaraki 305-0045 (JP); YOKOHAMA, Hiromitsu, Moriya-machi, Kitasoma-gun, Ibaraki 302- (JP); KOBAYASHI, Seiichi, Belmont, MA 02478 (US); SETO, Toshio, Sakae-cho, Ushiku-shi, Ibaraki 300-1233 (JP)
(74) Representative: Bannerman, David Gardner
(86) International application number: JP0003478
(87) International publication number: WO0072882

(57) **Abstract**

An agent for preventing preventing idiopathic thrombocytopenic purpura (ITP) comprising, as an active ingredient, a substance inhibiting interaction between gp39 on a T cell surface, which is a receptor mediating contact-dependent helper effector function, and CD40 on an antigen-presenting cell surface.

## Description

### TECHNICAL FIELD

The present invention relates to an agent for preventing idiopathic thrombocytopenic purpura (ITP).

### BACKGROUND ART

The interaction between a gp39 molecule on a CD4⁺ Th cell surface and a CD40 molecule on an antigen-presenting cell surface is essential for activation of the CD4⁺ Th cell by recognition of an antigen presented on an MHC class II antigen on the antigen-presenting cell surface by the CD4⁺ Th cell. When this interaction is inhibited by a gp39 antagonist, for example, an anti-gp39 antibody, T cell unresponsive, which is, T cell tolerance is induced in the CD4⁺ Th cell (WO95/06481).

Similarly, the interaction between a gp39 molecule on a CD4⁺ Th cell surface and a CD40 molecule on a B cell surface is essential for differentiation of B cell into an antibody-producing cell upon stimulation by an antigen. It is known that antibody production is blocked when this interaction is inhibited by a gp39 antagonist, for example, an anti-gp39 antibody (WO95/06480).

Since a gp39 antagonist inhibits both of immune responses of the CD4⁺ Th cell and the B cell, attempts are being made to apply gp39 antagonists to treatment of autoimmune diseases (systemic lupus erythematosus (SLE), ITP and so forth), in which pathological conditions are developed by immune responses to autoantigens.

### DISCLOSURE OF THE INVENTION

The inventors of the present invention expected from the action mechanism of the gp39 antagonist that immune unresponsiveness could be induced more easily by starting administration of the gp39 antagonist at a stage where an immune response begins to occur rather than starting the administration at a stage after the immune response was once established.

Many of autoimmune diseases are known to link with certain genes. It is suggested that idiopathic thrombocytopenic purpura (ITP) correlates to genetic polymorphism of a low affinity IgG receptor gene (Fcγ RIIA) (Williams et al., British Journal of Haematology 101: 779-82, 1998). It is inferred that it may become possible to predict an onset of ITP, if SNPs having a stronger correlation are identified through preparation of more organized single nucleotide polymorphisms (SNPs) maps and development of more advanced diagnosis techniques in future. Further, it is considered that, if a gp39 antagonist that induces immunological unresponsiveness is administered to a patient expected to develop ITP from a stage where an immune response to platelets begins to occur, the unresponsiveness can be more easily induced compared with the case where the antagonist is administered after an onset of ITP.

Furthermore, it is considered that, in an ITP patient in remission owing to administration of a steroid drug or the like, if the unresponsiveness can be induced by inhibiting the immune response to platelets, which is induced in an exacerbation period, through administration of a gp39 antagonist, the remission can be prolonged and thus high therapeutic value is provided.

The inventors of the present invention found that onsets of thrombocytopenia and anti-platelet autoantibody. production could be effectively prevented by administering an anti-gp39 antibody, which is a gp39 antagonist, to a male (NZW x BXSB) F₁ mouse, which genetically develops ITP-like pathological condition (thrombocytopenia and anti-platelet autoantibody production) with aging, prior to the onsets of the pathological conditions. Thus, they accomplished the present invention.

That is, the present invention provides an agent for preventing ITP containing, as an active ingredient, a substance inhibiting interaction between gp39 on a T cell surface, which is a receptor mediating contact-dependent helper effector function, and CD40 on an antigen-presenting cell surface.

The present invention also provides use of a substance inhibiting interaction between gp39 on a T cell surface, which is a receptor mediating contact-dependent helper effector functions, and CD40 on an antigen-presenting cell surface for manufacture of an agent for preventing ITP.

The present invention further provides a method of preventing onset of idiopathic thrombocytopenic purpura (ITP), which comprises administering a substance inhibiting interaction between gp39 on a T cell surface, which is a receptor mediating contact-dependent helper effector function, and CD40 on an antigen-presenting cell surface, to a patient expected to develop ITP.

The substance inhibiting the interaction between gp39 on the T cell surface, which is the receptor mediating contact-dependent helper effector function, and CD40 on the antigen-presenting cell surface is preferably an anti-gp39 antibody.

### BRIEF DESCRIPTION OF THE DRAWINGS

Fig. 1 shows a suppressive effect of an anti-gp39 antibody, MR1, on fatality of (NZW x BXSB) F₁ mice. ▲: MR1 (500 µg/mouse), △: Control group, #: p < 0.05 (Log-rank Test).
Fig. 2 shows a suppressive effect of MR1 on thrombocytopenia in (NZW x BXSB) F₁ mice. ▲: MR1 (500 µg/mouse), △: Control group, ∗: p < 0.05 (time course variance analysis). The values are shown as mean value ± standard error for surviving mice.
Fig. 3 shows a suppressive effect of MR1 on anti-platelet IgG antibody titer in plasma of (NZW x BXSB) F₁ mice. ▲: MR1 group (500 µg/mouse), △: Control group, ∗: p < 0.05 (time course variance analysis). The values are shown as mean value ± standard error for surviving mice.
Fig. 4 shows a suppressive effect of MR1 on platelet-binding anti-platelet IgG antibody titer in (NZW x BXSB) F₁ mice. ▲: MR1 (500 µg/mouse), △: Control group, ∗: p < 0.05 (time course variance analysis). The values are shown as mean value ± standard error for surviving mice.

### BEST MODE FOR CARRYING OUT THE INVENTION

Various embodiments of the present invention will be explained in detail below with respect to the following items.

### 1. gp39 antagonist

A gp39 antagonist is defined as a substance inhibiting interaction between gp39 on a T cell surface, which is a receptor mediating contact-dependent helper effector function, and CD40 on a antigen-presenting cell surface. The gp39 antagonists include not only substances interacting with gp39, but also substances interacting with CD40. The gp39 antagonist may be an antibody directed to gp39 (for example, monoclonal antibodies directed to gp39), a fragment of the antibody directed to gp39 (for example, Fab or F(ab')₂ fragment), a chimera antibody or a humanized antibody, soluble CD40 or soluble CD40L and a fragment thereof, or any other compounds inhibiting the interaction between gp39 and CD40.

The property of the gp39 antagonist for inhibiting the interaction between gp39 and CD40 can be determined based on, for example, if it inhibits binding of a labeled soluble CD40 to an activated helper T cell. The labeled soluble CD40 can be produced by preparing soluble CD40 using, for example, the method described in Example 1 in Japanese Patent Application Laid-open No. 6-220096 and labeling the soluble CD40 with an appropriate labeling substance, for example, a fluorescent substance, a radioisotope or the like. The activated helper T cell can be prepared by, for example, activation with an anti-CD3 antibody or the like according to the method described in Example 1 in Japanese Patent Application Laid-open No. 6-220096. Binding of the labeled soluble CD40 to the activated helper T cell can be evaluated by FACS using, for example, a fluorescence-labeled soluble CD40.

### 2. Anti-gp39 antibody

A mammal (for example, mouse, hamster or rabbit) can be immunized with a gp39 protein or a protein fragment thereof (for example, peptide fragments) in the form of an immunogen that induces immune responses in the mammal.

The gp39 protein can be obtained by allowing expression of an expression vector to which gp39 cDNA (Armitage et al., Nature, 357: 80-82, 1992; Hollembaugh et al., EMBO J., 11: 4313-4319, 1992) is inserted, in a host cell, for example, a bacterial or mammalian cell strain, and purifying the protein from a culture broth of the cell according to a standard method. The gp39 protein may be expressed as, for example, a fusion protein with GST or the like. A fusion protein with GST may be purified by using a glutathione column. The gp39 peptide can be synthesized by a known method (for example, F-moc or T-boc chemical synthesis) based on the amino acid sequence of gp39 (Armitage et al., Nature, 357: 80-82, 1992; Hollembaugh et al., EMBO J., 11: 4313-4319, 1992). Immunogenicity of the synthesized peptide can be improved by binding the peptide to an appropriate carrier, for example, KLH.

Antiserum can be obtained after immunization with the purified gp39 protein or the peptide fragment thereof together with an adjuvant. If desired, polyclonal antibodies can be isolated from the antiserum. To produce monoclonal antibodies, antibody-producing cells (lymphocytes) are recovered from an immunized animal and immortalized by fusing them with myeloma cells according to a standard cell fusion method to obtain hybridomas. This technique is an established method in this field and can be performed according to descriptions of an appropriate manual (Harlow et al., Antibodies: A Laboratory Manual, 1998, Cold Spring Harbor Laboratory). Further, monoclonal antibodies may also be prepared by other methods including the human B cell hybridoma method for producing human monoclonal antibodies (Kozbar et al., Immunol. Today, 4: 72, 1983), the EBV-hybridoma method (Cole et al., Monoclonal Antibody in Cancer Therapy, 1985, Allen R. Bliss, Inc., pages 77-96), screening of combinatorial antibody library (Huse et al., Science, 246: 1275, 1989) and so forth.

The antibodies referred to in the present specification include fragments of antibodies specifically binding to gp39, for example, Fab and F(ab')₂ fragments.

Monoclonal antibodies prepared by using an animal other than human, e.g., mouse monoclonal antibodies prepared by using mouse as an immunized animal, are often recognized as heterogenous proteins when administered to human, and therefore an immune response to the antibodies may be often induced. A solution to this problem is provided by chimera antibodies, that is, antibodies having an antigen-binding region derived from a mouse monoclonal antibody and other regions derived from a human antibody. The antibodies used in the present invention also include such chimera antibodies. Examples of the chimera antibodies include chimera antibodies using the whole variable regions of a mouse monoclonal antibody as an antigen-binding region (Morrison et al., Proc. Natl. Acad. Sci. USA, 81: 6851, 1985; Takeda et al., Nature, 314: 452, 1985), chimera antibodies utilizing a combination of a human-derived framework region and a hypervariable region derived from a mouse monoclonal antibody as an antigen-binding region (Teng et al., Proc. Natl. Acad. Sci. USA, 80: 7308-12, 1983; Kozbar et al., Immunol. Today, 4: 7279, 1983) and so forth. However, the present invention is not limited to these.

The agent for preventing ITP of the present invention can be administered to a patient expected to develop ITP (including ITP patients in remission owing to administration of a steroid drug or the like) to prevent an onset (as well as exacerbation) of IPT.

The agent for preventing ITP of the present invention can be administered by a usual method such as injection (subcutaneous, intravenous or the like).

The form of the agent for preventing ITP is appropriately selected depending on the administration method. Examples of pharmaceutical compositions suitable for use as injection include, for example, sterilized aqueous solutions (when the substance is water-soluble), dispersions, sterilized powders for immediately preparing sterilized injection solutions or dispersions. The pharmaceutical compositions suitable for use as injection must be sterilized and has sufficient fluidity for easy syringe operation in any case. The compositions must be stable under production and storage conditions and be protected from actions of contaminated microbes such as bacteria and fungi. A carrier may be a solvent or a dispersion medium, for example, water, ethanol, polyol (for example, glycerol, propylene glycol, polyethylene glycol etc.), an appropriate mixture thereof or the like. Appropriate fluidity can be maintained by using a coating such as one composed of lecithin, or maintaining a desired particle diameter or using a surfactant in case of dispersion. The protection from actions of microbes can be achieved by various antibacterial agents or antifungal agents, for example, paraben, chlorobutanol, phenol, ascorbic acid, thimerosal and so forth. In many cases, it is preferable to add isotonic agents, for example, saccharides and polyalcohols including mannitol and sorbitol, sodium chloride and so forth to the composition. Sustained absorption of compositions for injection can be attained by adding substances for retarding the absorption, for example, aluminum monostearate, gelatin or like to the compositions.

The solutions for injection can be prepared by mixing an active compound (gp39 antagonist) in a desired amount and, if required, one of the aforementioned ingredients or a combination thereof in an appropriate solvent and then sterilizing the mixture by filtration. In general, dispersions are prepared by mixing an active compound with a base dispersion medium and a sterilized medium containing other required ingredients selected from the above. In case of using sterilized powder for preparing sterilized injection solution, preferable methods are vacuum dehydration or lyophilization, and powder of the active ingredient and desired additional ingredients sterilized by filtration beforehand can be obtained.

Doses of the agent for preventing ITP are amounts sufficient to prevent onset of ITP and can vary depending on age, sex, susceptibility to the substance, administration method, clinical history and so forth of patients.

### EXAMPLES

The present invention will be described in more detail with reference to the following examples, but the present invention is not limited to these.

The anti-gp39 antibody MR1 (Noelle et al., Proc. Natl. Acad. Sci. USA, 89: 6550-54, 1992) used in the following examples is a monoclonal antibody directed to a mouse gp39 prepared in a hamster and is available from PharMingen (Catalog No.: PM-09020D or PM-09021D). The MR1-producing cell is available from American Type Culture Collection (ATCC) (ATCC No.: HB-11048).

In the (NZW x BXSB) F₁ mice used, autoantibodies directed to platelets emerge in blood after the age of 3 months and remarkable decline in the number of platelets is observed with aging (Ikehara, Metabolism, 26: 169-179, 1989). Therefore, they are regarded as an ITP model (Adachi et al., Immunobiol., 198: 451-64, 1998). These mice are available from Japan SLC.

### Example 1: Suppressive effect of MR1 on fatality of (NZW x BXSB) F₁ mice

A suppressive effect of MR1 on fatality of (NZW x BXSB) F₁ mice was examined. MR1 (Noelle et al., Proc. Natl. Acad. Sci. USA, 89: 6550-54, 1992) was purified from a culture supernatant of MR1-producing cells presented by Dr. Noelle by using protein A, and used. MR1 and hamster IgG as a negative control were each intraperitoneally administered to the mice once a week at a dose of 500 µg/200 µL/administration from the age of 5-week old through 16-week old.

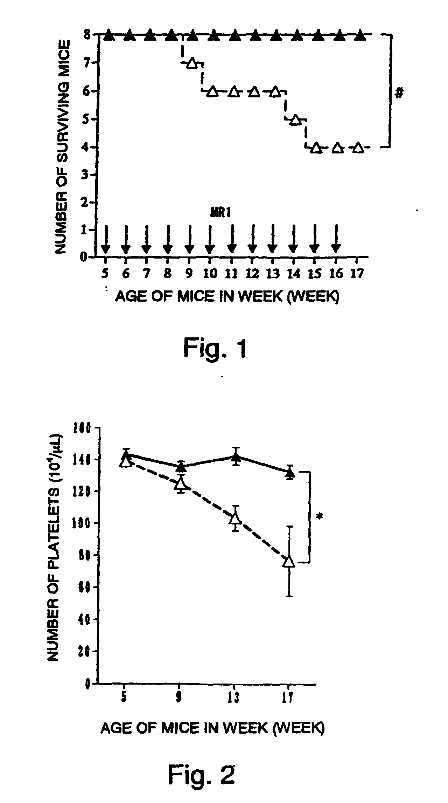

As a result, in the hamster IgG-administered (NZW x BXSB) F₁ control group, deaths were observed from the age of 9-week old. In this group, the survival rate lowered to 50% by the age of 15-week old and was maintained at this level until the age of 17 weeks at which the test was finished. In the MR1-administered group, on the other hand, no death was observed at all during the test period. Improvement of the survival rate of this group was statistically significant as compared with that of the control group (p < 0.05, Fig. 1).

### Example 2: Suppressive effect of MR1 on thrombocytopenia in (NZW x BXSB) F₁ mice

A suppressive effect of MR1 on thrombocytopenia in (NZW x BXSB) F₁ mice was examined. MR1 and hamster IgG as a negative control were each intraperitoneally administered to mice once a week at a dose of 500 µg/200 µL/administration from the age of 5-week old through 16-week old. About 300 µL of blood was collected from the fundus oculi into an EDTA-treated tube by using a heparin-treated hematocrit capillary tube at the ages of 5, 9, 13 and 17-week old under etherization. The number of platelets in the collected blood was measured by using a comprehensive hematological test apparatus system, Technicon H·1™ system (Bayer Corporation, Tarry Town, NY).

As a result, the number of platelets in the mice in the hamster IgG-administered control group showed 26% and 45% declines at the ages of 13- and 17-week old, respectively, compared with that at the age of 5-week old. In the MR1-administered group, on the other hand, almost no decline in the number of platelets was observed even at the age of 17-week old at which the test was finished. There was a statistically significant difference between the MR1-administered group and the control group. (p < 0.05, Fig. 2)

### Example 3: Suppressive effect of MR1 on anti-platelet IgG antibody titer in plasma of (NZW x BXSB) F₁ mice

A suppressive effect of MR1 on the anti-platelet IgG antibody titer in plasma of (NZW x BXSB) F₁ mice was examined. The plasma isolated from the blood collected in Example 2 was allowed to react with mouse platelets fixed on a 96-well plate with glutaraldehyde, and then the anti-platelet antibody titer in the plasma was measured by ELISA utilizing detection with peroxidase-labeled anti-mouse IgG. The antibody titer of samples (A.U./ml) was calculated based on an antibody titer of a serum of a syngenic mouse of 35-week old used as a standard serum, which was taken as 1000 U/ml.

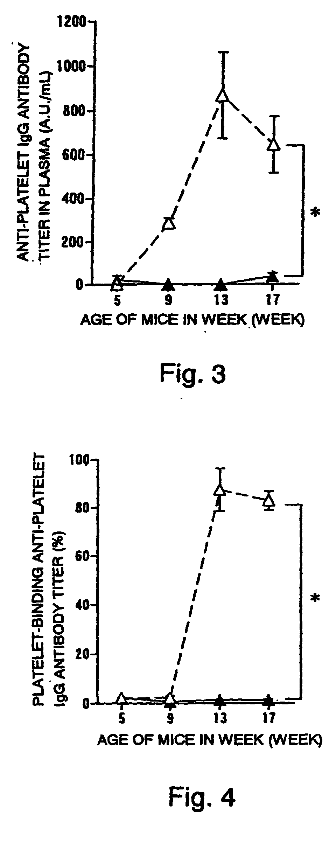

As a result, the anti-platelet IgG antibody titer in plasma of the hamster IgG-administered control group increased from 9-week old and reached the peak at 13-week old. MR1 almost completely inhibited the increase of the autoantibodies in blood until 17-weeks old at which the test was finished. A statistically significant difference was observed between the MR1-administered group and the control group (p < 0.05, Fig. 3).

### Example 4: Suppressive effect of MR1 on platelet-binding anti-platelet IgG antibody titer in (NZW x BXSB) F₁ mice

A suppressive efffect of MR1 on (NZW x BXSB) F₁ mouse platelet-binding anti-platelet IgG antibody titer was examined. The platelet-binding anti-platelet antibody titer was obtained as follows. Platelets separated from the blood collected in Example 2 were allowed to react with FITC-labeled anti-mouse IgG (Fc), and the fluorescent dye bound to the platelets was measured by using a FACScan™ flow cytometer (Beckton-Dickinson, San Jose, CA). Ratio of platelets exhibiting higher fluorescent intensity compared with platelets of BALB/c mice (negative control) was calculated for each individual, and the obtained value was used as a platelet-binding anti-platelet IgG antibody titer.

As a result, in the mice of the hamster IgG-administered control group, increase of platelet-binding anti-platelet IgG antibodies was observed after 13-week old. In these mice, the IgG antibodies were detected for about 87% and about 83% of platelets on average at the ages of 13-week old and 17-week old, respectively. In the MR1-administered group, on the other hand, no obvious increase in this parameter was observed until 17-week old at which the test was finished. A statistically significant difference was observed between the MR1-administered group and the control group (p < 0.05, Fig. 4).

### INDUSTRIAL APPLICABILITY

According to the present invention, when onset of idiopathic thrombocytopenic purpura (ITP) is expected, the onset of ITP can be prevented by prophylactic administration of a gp39 antagonist.

## Claims

1. An agent for preventing idiopathic thrombocytopenic purpura (ITP) comprising, as an active ingredient, a substance inhibiting interaction between gp39 on a T cell surface, which is a receptor mediating contact-dependent helper effector function, and CD40 on an antigen-presenting cell surface.

2. The agent for preventing ITP according to Claim 1, wherein the substance is an anti-gp39 antibody.
